(19) **Europäisches Patentamt**
**European Patent Office**
**Office européen des brevets**

(11) **EP 4 400 188 A1**

(12) **EUROPEAN PATENT APPLICATION**
published in accordance with Art. 153(4) EPC

(43) Date of publication:
**17.07.2024 Bulletin 2024/29**

(21) Application number: **22866815.8**

(22) Date of filing: **06.09.2022**

(51) International Patent Classification (IPC):
**B01D 11/02** [(2006.01)]  **A23L 33/105** [(2016.01)]
**A01N 65/08** [(2009.01)]  **C05F 5/00** [(2006.01)]
**A01K 67/033** [(2006.01)]

(52) Cooperative Patent Classification (CPC):
Y02P 20/145

(86) International application number:
**PCT/ES2022/070561**

(87) International publication number:
**WO 2023/037027 (16.03.2023 Gazette 2023/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.09.2021 ES 202130845**

(71) Applicants:
• **Consejo Superior De Investigaciones Científicas - CSIC**
**41013 Sevilla (ES)**
• **Universidad de Sevilla**
**41013 Sevilla (ES)**

(72) Inventors:
• **GARCÍA BORREGO, Aranzazu**
**41013 Sevilla (ES)**
• **ABIA GONZÁLEZ, Rocío**
**41013 Sevilla (ES)**
• **OROZCO PONCELA, Alejandro**
**41013 Sevilla (ES)**
• **RODRÍGUEZ JUAN, Elisa María**
**41013 Sevilla (ES)**
• **CARMONA MORENO, Inmaculada**
**41013 Sevilla (ES)**
• **AGUIRRE JIMÉNEZ, María Itziar**
**41013 Sevilla (ES)**

(74) Representative: **Pons**
**Glorieta Rubén Darío 4**
**28010 Madrid (ES)**

(54) **METHOD FOR OBTAINING A PHENOLIC EXTRACT FROM ALPEORUJO (OLIVE OIL BY-PRODUCT)**

(57)    The present invention relates to a method for obtaining an extract rich in phenolic compounds from fresh alpeorujo (olive oil by-product), using an acid-based natural deep eutectic solvent (NADES). The invention also relates to the use of the phenolic extract as a nutraceutical, food additive and phytosanitary product, particularly for the treatment of microbial diseases. In addition, the invention relates to alpeorujo extracted for compost and vermicompost, particularly as fertiliser and/or organic soil amendment.

EP 4 400 188 A1

## Description

The present invention relates to a method for obtaining an extract rich in phenolic compounds from fresh alpeorujo (olive oil by-product), using an acid-based natural deep eutectic solvent (NADES). The invention also relates to the use of the phenolic extract as a nutraceutical, food additive and phytosanitary product, particularly for the treatment of microbial diseases. In addition, the invention relates to alpeorujo extracted for compost and vermicompost, particularly as fertiliser and organic soil amendment.

## STATE OF THE ART

Alpeorujo is the main by-product that is obtained from the olive oil extraction process using the two-phase centrifugation system, in which olives are extracted in the oil mill through the grinding, mixing and centrifugation processes to produce, on the one hand, an oil phase, and on the other, a single by-product called alpeorujo. With this system, approximately 20% of oil is produced and 80% of by-product is generated.

The olive is made up of vegetation water, oil, skin, pulp and a pit. After 2-phase extraction, essentially all the components of the olive, except most of its oil, become alpeorujo. Thus, alpeorujo is a heterogeneous substance made up of the skin, pulp, pit of the olive and a part of its oil, with high humidity ranging between 65% and 75%. It has a semi-solid consistency, is expensive to manage, especially due to its phytotoxicity (E. Benitez, E., Sainz, H., Melgar, R., Nogales, R. 2002, Vermicomposting of a lignocellulosic waste from olive oil industry: A pilot scale study. Waste Manage Res. 134-142), difficult biological degradation (Venieri, D., Rouvalis, A., Iliopoulou-Georgudaki, J. 2010. Microbial and toxic evaluation of raw and treated olive mill wastewaters. J Chem Tech & Biotech, Vol. 85, 1380-1388) and high production thereof (greater than 2.4 million tons worldwide only in the 2020/2021 olive campaign (IOC. Internationaloliveoil.org) which is concentrated in the 4-5 months of the campaign, and causes the alpeorujo to have a strong environmental impact, turning this by-product into an economic burden for the olive industry.

However, alpeorujo is rich in bioactive compounds, in particular phenolic compounds that are very abundant in olives and after the extraction of olive oil remain retained up to 98% in the alpeorujo (S. Demerche, M. Nadour, C. Larrocheb, F. Moulti-Matia, P. Michaudb. (2013). Olive mill wastes: Biochemical characterizations and valorization Strategies. Process Biochem vol 48. 1532-1552). Furthermore, alpeorujo contains other healthy and nutritionally valuable substances such as lipids, carbohydrates and proteins.

Phenolic compounds can have neuroprotective effects in various pathologies of the nervous system through controlling oxidative stress, inflammation, apoptosis and mitochondrial dysfunction. According to the most recent scientific literature, dietary intake of phenolic compounds attenuates oxidative stress and reduces the risk of related neurodegenerative diseases, such as Alzheimer's disease, Parkinson's disease, strokes, multiple sclerosis and Huntington's disease. Moreover, at a peripheral level, they act as antioxidants, defending tissues against oxidative damage and eliminating free radicals. [Polyphenols from Food and Natural Products: Neuroprotection and Safety. Silva RFM, Pogačnik L. Antioxidants (Basel). 2020 Jan 10;9(1)].

Many synthetic antioxidants have been used to slow the oxidation process, particularly in food systems (Shahidi, F. and Naczk, M. 2004. Phenolics in Food and Nutraceuticals, 576 Boca Raton, FL: CRC Press.). However, the use of synthetic antioxidants is under strict regulation due to their potential health hazard in promoting carcinogenesis, as well as the general rejection of synthetic food additives by consumers. Tocopherols and ascorbic acid or their derivatives that are used as alternatives to synthetic antioxidants are much less effective antioxidants. (Emerging Role of Phenolic Compounds as Natural Food Additives in Fish and Fish Products. Sajid Maqsood, Soottawat Benjakul & Fereidoon Shahidi. Critical Reviews in Food Science and Nutrition. Volume 53, 2013 - Issue 2).

Moreover, there is a growing demand from the agri-food industry for antimicrobials of natural origin associated with the restrictions of European regulations on plant protection products and the regulation on their sustainable use (Directive 2009/128/EC. Regulation (EC) No. 1107/2009 (Directive 2009/128/EC) associated with the risks to health and the environment that they entail (toxicity, long-term persistence, promotion of pathogen resistance). Consumers demand foods that are free of synthetic chemical residues which are associated with harmful health effects (Balasubramanian, P., Karthickumar, P. 2017. Biofertilizers and biopesticides: A holistic approach for sustainable agriculture. Sustainable utilization of natural resources 256-284; Kulkarni, S. 2016. Sustainable agrochemicals for conservation of agriculture and climate change. Conservation agriculture: An approach to combat climate change in indian himalaya pp. 135-157).

The antimicrobial activity of phenolic compounds has been described, including phenols from olives and olive oil (Brenes, M., Medina, E., Garcia, A., Romero, C., de Castro, A. 2010. Olives and olive oil compounds active against pathogenic microorganisms, Health and disease prevention, 109, 1013-1019).

Alpeorujo is rich in these compounds. Once stored or transformed, chemical and biological alterations occur in the same (Sánchez, P., Ruiz, M.V. 2006. Production of pomace olive oil. Grasas y aceites 57, 47-55) that reduce or modify its phenolic content but still exhibit antimicrobial action against a wide spectrum of microorganisms, among them phytopathogenic bacteria (Medina, E., Romero, C., Santos, B., Castro, A., Garcia, A., Romero, F., Romero, F., Brenes, M. 2011. Antimicrobial activity of olive solutions from stored alpeorujo against plant pathogenic microorganisms. J. Agric. Food Chem. 59, 6927-6932).

The agri-food industry and the scientific community also seek to obtain bioactive compounds of natural origin, which are obtained using non-toxic, biodegradable and low-cost extractants as an alternative to conventional organic solvents that are used extensively and which mostly do not comply with these premises.

Solvents with high extractive capacity of bioactive substances are required, which produce low-toxicity extracts that can be used in the agri-food industry and have a low environmental impact.

Natural deep eutectic solvents (NADES) are made up of mixtures of natural substances present in living organisms and they can be an alternative to conventional solvents depending on their formulation. NADES can be made up of very different combinations of substances. NADES have typically been formulated to extract bioactive substances based on quaternary ammonium salts such as choline chloride, associated with other components such as sugars, alcohols, or amino acids, among others (Dai, Y., Witkamp, G. J., Verpoorte, R., Choi, Y. H. 2013. Natural Deep Eutectic Solvents as a New Extraction Media for Phenolic Metabolites in Carthamus tinctorius. L. Anal. Chem. 85, 6272-6278). However, these NADES can exhibit toxicity against cells and animals, which limits their use as a food additive or nutraceutical. Radoševic, K., Bubalo, M. C., Srcek, V. G., Grgas, D., Dragicevic, T. L., Redovnikovic, I. R. 2015. Evaluation of toxicity and biodegradability of choline chloride based deep eutectic solvents. Ecotoxicol Environ Saf. 112, 46-53. Furthermore, their composition contains chlorine which makes them environmentally toxic.

Different eutectic solvents have been used to extract phenols from olive oil and alpeorujo based on ammonium salts and other components (Garcia, A., Rodriguez-Juan, E., Rodríguez-Gutiérrez, G., Rios, J. J., Fernández-Bolaños, J. 2016. Extraction of phenolic compounds from virgin olive oil by deep eutectic solvents (DESs). Food Chem. 197, 554-561. Garcia Borrego, A., Rodriguez-Juan, E., Fernandez-Bolaños, J. 2017. Extraction of phenolic compounds from olive pomace by deep eutectic solvents (DESs), Trends in Green chem. 3:3).

Alpeorujo generated in the oil mill, is stored in evaporation ponds for a variable period of time, from months to years. During this period, numerous chemical and biological transformations occur in the same which convert this by-product into a different substance from fresh alpeorujo obtained in the oil mill, especially due to the degradation of its phenolic content, formation of substances derived from fermentation, chemical alteration and water loss. After storage, alpeorujo is used for different uses, such as obtaining its residual oil (Sánchez, P., Ruiz, M.V. 2006. Production of pomace olive oil. Grasas y aceites 57, 47-55). For this process, it is dried at high temperature, reducing its water content from 65-70% to 8-10%, wherein drying is a necessary process to allow residual oil to be extracted using an organic solvent, mainly hexane, which is nonpolar and immiscible in water, meaning that it is not extractive in aqueous matrices. Under temperature and vacuum conditions, oil is extracted from the dry alpeorujo. Subsequently, the dried and extracted alpeorujo is desolvated (elimination of the solvent, hexane, which is volatile with a boiling point of 69°C), leaving a residue of the dry and oil-free alpeorujo that is called "de-oiled extraction cake". This residue has been transformed by temperature, the vacuum, and the organic solvent, meaning that it is very different chemically and physically from fresh alpeorujo obtained in the oil mill.

Both the alpeorujo stored in ponds and the alpeorujo dried and extracted using an organic solvent to obtain its oil are physically-chemically and biologically different from fresh alpeorujo obtained from the oil mill and can be recycled through different biotechnological methods, among them, composting (Tortosa, G., Albuquerque, J.A., Ait-Baddi, G., Cegarra, J. 2012. The production of commercial organic amendments and fertilisers by composting of two-phase olive mill waste ("alpeorujo"). Journal of Cleaner Production 26, 48-55) and vermicomposting.

Vermicomposting facilitates the transformation of organic waste through low-cost processes in which microorganisms and worms intervene, giving rise to a product, vermicompost, useful for agriculture as a fertiliser and/or soil conditioner (Melgar, R., Benitez, E., Nogales, R., 2009. Bioconversion of wastes from olive oil industries by vermicomposting process using the epigeic earthworm Eisenia andrei. Journal of Environmental Science and Health, Part B, 44, 488-495. Nogales, R., Romero, E., Fernandez-Gómez, M. J. 2014. Vermicompost: Processes, products and applications. Mundi-Prensa, Madrid).

Therefore, it would be desirable to have a method that would allow obtaining a bioactive and antimicrobial extract from fresh alpeorujo using a natural deep eutectic solvent (NADES), and through processes such as vermicomposting that would allow for the sustainable management of olive oil production with zero waste.

## DESCRIPTION OF THE INVENTION

In a first aspect, the present invention relates to a method for obtaining a phenolic extract from alpeorujo that comprises the following steps:

    i. preparing the natural deep eutectic solvent (NADES 4) selected from malic acid and sucrose in a 1:1 w/w ratio, containing between 8% and 35% by weight of water; and
    ii. adding NADES 4 from step (i) to fresh alpeorujo in an alpeorujo: NADES ratio between 5:1 and 1:5 w/w, stirring for a time of between 10 minutes and 6 hours, at a temperature between 20°C and 90°C, and centrifuging between 1000 rpm and 10,000 rpm to separate the phenolic extract phase with the solvents of the alpeorujo extracted (ALE) with NADES 4.

The "fresh alpeorujo" to which the invention relates is the alpeorujo as such, in other words, the one obtained directly from the oil mill, without any treatment or transformation.

In another embodiment, the invention relates to the method as defined above, wherein step (ii) is repeated one or more times, preferably once or twice, and more preferably once.

In another embodiment, the invention relates to the method as defined above, wherein in step (i) of preparing the natural deep eutectic solvent (NADES 4), the water content is between 10% and 30% by weight of water, and preferably 10% and 25% by weight of water, and more preferably 19% by weight of water.

In another embodiment, the invention relates to the method as defined above, wherein in step (ii), the ratio to the NADES of step (i) and alpeorujo is 1:1 w/w.

In another embodiment, the invention relates to the method as defined above, wherein step (ii) is carried out at a temperature between 30°C and 50°C, and preferably 40°C.

In another embodiment, the invention relates to the method as defined above, wherein step (ii) is carried out by centrifuging between 1000 rpm and 10,000 rpm.

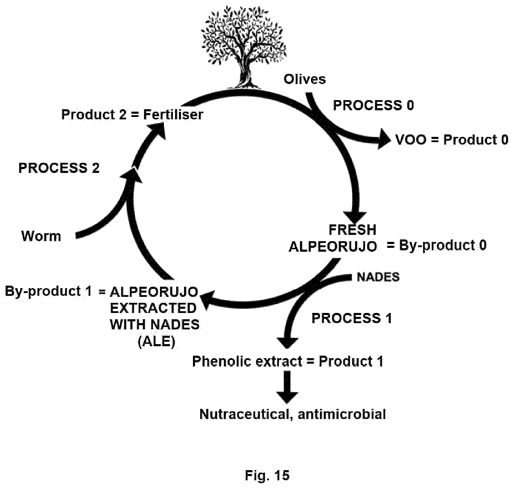

After extracting alpeorujo using NADES 4 (made up of malic acid/sucrose as defined above), an extract rich in polyphenols that we call "phenolic extract", and a residue that we call "alpeorujo extracted with NADES 4 (ALE)" are obtained. Taking into account circular economy principles (see figure 15), ALE can be transformed through processes with essentially zero residue.

Various biological processes may be used, among which composting and vermicomposting is worth noting. In this case, work with vermicomposting has been carried out, including a prior conditioning treatment of the material that could be considered pre-compost. Work has been carried out with worms of the species *Eisenia fetida.* This process of biological transformation is justified by taking into account the difficulty in using the extracted alpeorujo (ALE) as it is obtained. The alpeorujo extracted with NADES 4 has very different physical and chemical characteristics from fresh alpeorujo. Fresh alpeorujo is only made up of the components of the olive minus the oil that has been extracted in the oil mill, leaving 3% to 10% of residual oil (expressed as wet oil content, WOC%), and it is slightly acidic (pH $\approx$ 5.5), with a humidity of 55% to 70%, so it is semi-solid and has a high phenolic content. When extracting fresh alpeorujo with the NADES 4 solvent, this by-product is transformed. NADES 4 is a eutectic solvent with very different characteristics from conventional solvents, it is an acidic gel (pH 0.71) formed by malic acid, sucrose and water, it is transparent, dense ($\rho \approx 1.25$) and viscous. It has low volatility, a decomposition point greater than 135°C and a low freezing point. After extracting the phenolic compounds from fresh alpeorujo with NADES 4, up to 8% of it remains closely linked to the extracted alpeorujo and cannot be separated from the same, forming part of its structure and leaving the alpeorujo enriched in malic acid and sucrose combined in NADES 4. The alpeorujo extracted with NADES 4 is different from the starting fresh alpeorujo; it is acidic (pH 2.5), contains up to 2% residual oil (WOC%), its humidity ranges from 20-30% since it has been dehydrated by extraction with NADES 4, its consistency is viscous due to its residual NADES 4 content and the phenolic content has also been eliminated. Its chemical properties, especially its acidity, and physical factors (especially its high viscosity and poor aeration) make the alpeorujo extracted with NADES 4 a product that is difficult to transform through biological mechanisms. The composting and vermicomposting processes used in this invention facilitate its transformation. Circular economy processes are implemented that make this invention an essentially zero waste process based on the by-product of olive oil production.

Another aspect of the invention relates to the phenolic extract from alpeorujo obtained by the method described above.

The phenolic extract of the present invention is a set of bioactive compounds that may have anti-inflammatory and antioxidant capacity.

Thus, another aspect of the invention relates to the use of the phenolic extract defined above as a nutraceutical or food additive.

Another aspect of the invention relates to the use of the phenolic extract defined above as a phytosanitary product.

In another embodiment, the invention relates to the use of the phenolic extract defined above as a phytosanitary product in the treatment of microbial plant diseases, preferably for the treatment of microbial plant diseases caused by bacteria of the genera *Erwinia, Pseudomonas, Xanthomonas* and *Rhizobium.*

Another aspect of the invention relates to alpeorujo extracted with NADES 4 (ALE) obtained by the method described above.

Another aspect of the invention relates to the use of alpeorujo extracted with NADES 4 (ALE) defined above as an organic supplement for compost and vermicompost.

In another embodiment, the invention relates to the use of alpeorujo extracted with NADES 4 (ALE) defined above as an organic supplement for vermicompost, characterised in that ALE treatment is carried out with worms of the species *Eisenia fetida.*

The vermicompost thus obtained gives rise to a useful product as a fertiliser or organic soil conditioner.

Another aspect of the invention relates to the natural deep eutectic solvent (NADES 4) selected from malic acid and sucrose in a 1:1 w/w ratio.

In another embodiment, the invention relates to the natural deep eutectic solvent (NADES 4) defined above where the water content is between 8% and 35% by weight of water, preferably between 10% and 25% by weight of water, and more preferably 19% by weight of water.

Throughout the description, the term "fertiliser" refers to a product used in agriculture or gardening that, due to its nutrient content, facilitates plant growth (Royal Decree 506/2013)

The term "organic soil conditioner" refers to a product from carbon materials of plant or animal origin, fundamentally used to maintain or increase the organic matter content of soil, improve its physical properties and also improve its chemical or biological properties or activity (Royal Decree 506/2013). The present invention relates to alpeorujo extracted with NADES (ALE) as a carbon material of plant origin.

The term "alpeorujo extracted with NADES 4" refers to the residue of fresh alpeorujo after extraction using the natural deep eutectic solvent (NADES) of bioactive compounds, mainly phenolic compounds; therefore, it contains a small amount of the same.

The term "nutraceutical" (bioactive agent) refers to products of natural origin found in the alpeorujo extract obtained with NADES 4, which can be administered as such in concentrated form or added to a natural food and which are beneficial for health and have a preventive and/or therapeutic capacity. Examples include, among other things, phytosterols, phenolic compounds, etc.

The term "food additive" refers to products of natural origin found in the alpeorujo extract that, although they are not consumed as food by themselves, are added to food during production and fulfil an important technological function, collaborating in the crucial task of obtaining safe, quality food.

The term "microbial disease" refers to the response of plant cells and tissues to pathogenic microorganisms that cause damage to the physiology or integrity of the plant and may cause its deterioration and even its death. Examples of bacterial plant diseases include, among others, the "fire blight" caused by *Erwinia amylovora* on pome or stone fruit trees in rosaceae such as the apple tree, medlar tree, quince tree, among others, and that causes the death of the plant; "tuberculosis" in oleaceae, including the olive tree, producing tumours, galls or warts with loss of vigour and productivity of the crop, the infection affecting the organoleptic properties and stability of the oil caused by *Pseudomonas savastanoi*; necrosis, galls, chancres, which destroys the infected tissue, depleting the fruit plant such as the pear tree, mango tree, citrus trees, other horticultural and ornamental crops caused by *Pseudomonas siryngae*; tumours in the neck of the plant, reducing the productivity and quality of crops such as vineyards, pome fruit and walnut trees caused by *Rhizobium radiobacter,* or the wilting of horticultural and fruit crops caused by *Xanthomonas campestris.*

Thus, in the present invention, a low toxicity acid-based NADES is used for the first time to obtain bioactive substances, especially phenolic compounds, of fresh olive alpeorujo and a new by-product of alpeorujo extracted with NADES 4 is generated.

After extracting alpeorujo with NADES 4, a new by-product is generated that is different from the fresh alpeorujo obtained in the oil mill, the alpeorujo stored in evaporation ponds or the alpeorujo extracted to obtain its residual oil by physical (centrifugation) and/or chemical methods with conventional organic solvents (mainly hexane); this alpeorujo extracted with hexane has been previously stored, dried and subjected to vacuum and temperature. The alpeorujo extracted with NADES 4" is a new substance that is difficult to handle due to its physicochemical characteristics, its high viscosity, low pH and poor aeration. A methodology has been developed for the vermicomposting of this new by-product. In this way, the extracted alpeorujo (ALE) is recycled and the design of zero waste processes is facilitated in which the materials and nutrients contained in the product return to the agricultural parcels in the format of fertilisers and/or soil conditioners.

Therefore, the present invention represents a turning point in the comprehensive use of the by-product of olive oil production, alpeorujo, which allows for the comprehensive use of the same to obtain, on the one hand, its bioactive compounds using extractive acidic eutectic mixtures consisting of food-grade and low-toxicity substances that generate bioactive extracts, and on the other hand, the by-product of this extraction, extracted alpeorujo, is transformed to be reincorporated into the soil. With this invention, it contributes to the comprehensive use of alpeorujo and the sustainable management of olive oil production with zero waste.

Throughout the description and the claims, the word "comprises" and its variants do not intend to exclude other technical features, additives, components or steps. For those skilled in the art, other objects, advantages and features of the invention may be partially deduced from both the description and the embodiment of the invention. The following examples and figures are provided by way of illustration and are not intended to limit the present invention.

**BRIEF DESCRIPTION OF THE FIGURES**

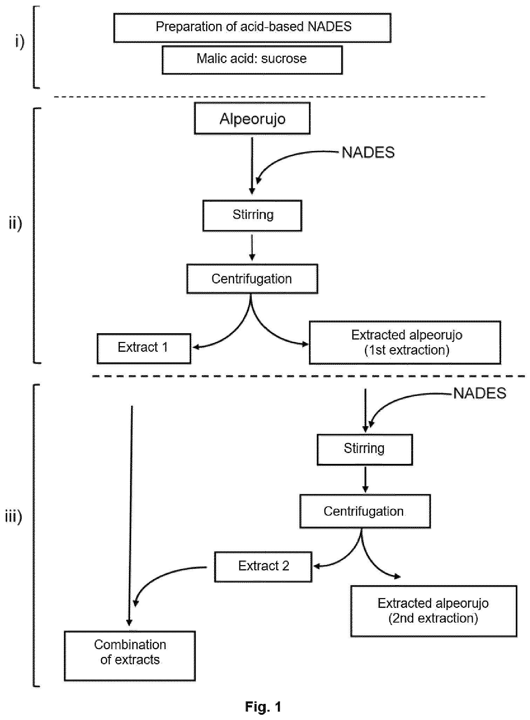

**Fig. 1.** Summary diagram of the phenolic extraction method.

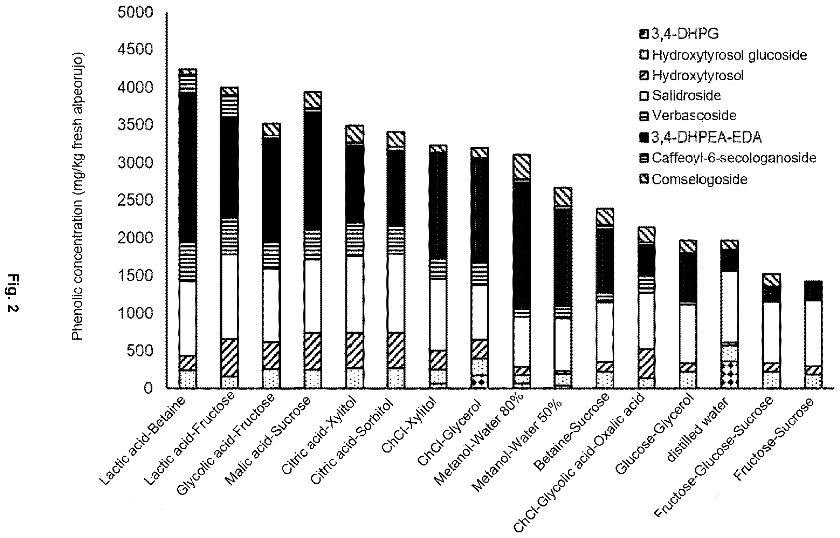

**Fig. 2.** Concentration of individual phenolic compounds (mg/kg) extracted from fresh alpeorujo of the Picual variety using different natural deep eutectic solvents, aqueous mixtures of methanol and water. The solvents correspond to those listed in table 1. Analyses were performed by HPLC-UV with confirmation of structures by means of HPLC-MS. (n=2).

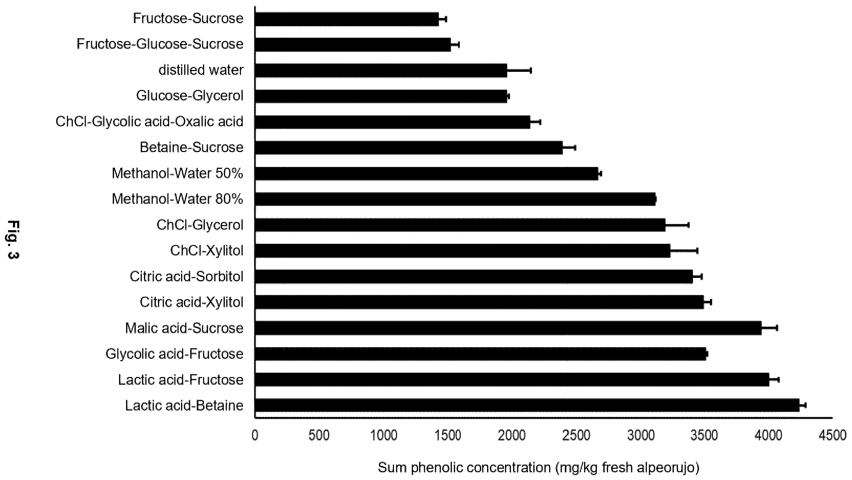

**Fig. 3.** Sum concentration of phenolic compounds extracted (mg/kg) from fresh alpeorujo of the Picual variety using different eutectic solvents, aqueous mixtures of methanol and water. The solvents correspond to those listed in table 1. Analyses were performed by HPLC-UV with confirmation of structures by HPLC-MS (n=2).

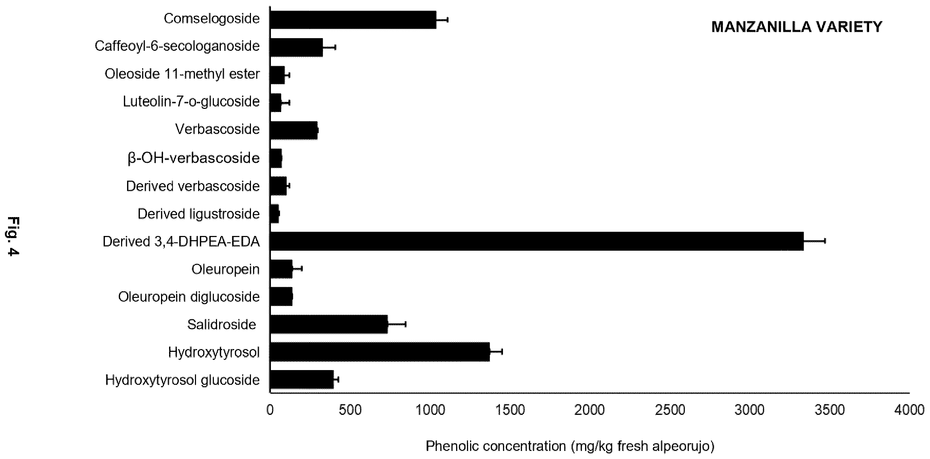

**Fig. 4.** Concentration of individual phenolic compounds (mg/kg) extracted from fresh alpeorujo of the Manzanilla variety using NADES 4. Analyses were performed by HPLC-UV with confirmation of structures by HPLC-MS (n=2).

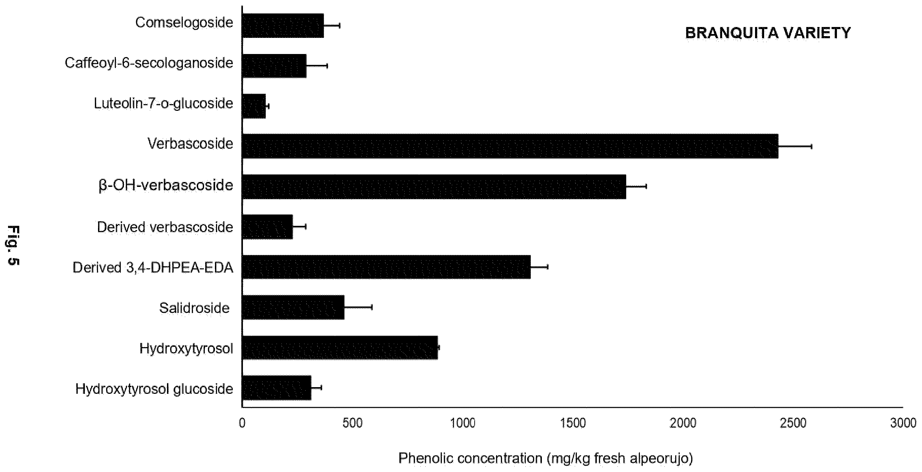

**Fig. 5.** Concentration of individual phenolic compounds (mg/kg) extracted from fresh alpeorujo of the Branquita variety using NADES 4. Analyses were performed by HPLC-UV with confirmation of structures by HPLC-MS (n=2).

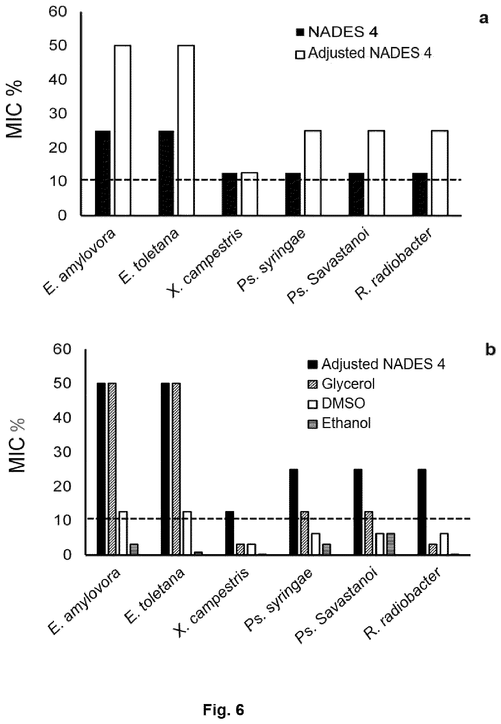

**Fig. 6.** Bactericidal effect of NADES 4 on phytopathogenic microorganisms expressed as a minimum inhibitory concentration (MIC) percentage, (**6a**) NADES 4 at original acidic pH and at adjusted pH 5, 5-6, (**6b**), NADES 4 at adjusted pH, glycerol, DMSO and ethanol, on *Erwinia amylovora* CECT 222, *Erwinia toletana* CECT 5263, *Xanthomonas campestris* CECT 97, *Pseudomonas syringae* CECT 4429, *Pseudomonas savastanoi* CECT 5019 and, *Rhizobium radiobacter* CECT 4119. (n=3). The dotted line indicates the value below which toxicity is considered.

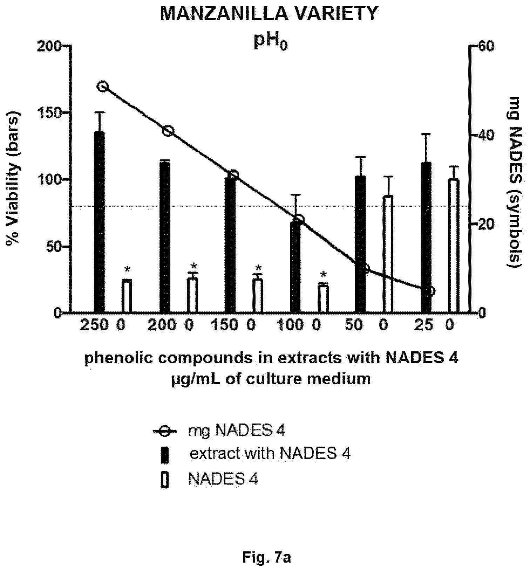

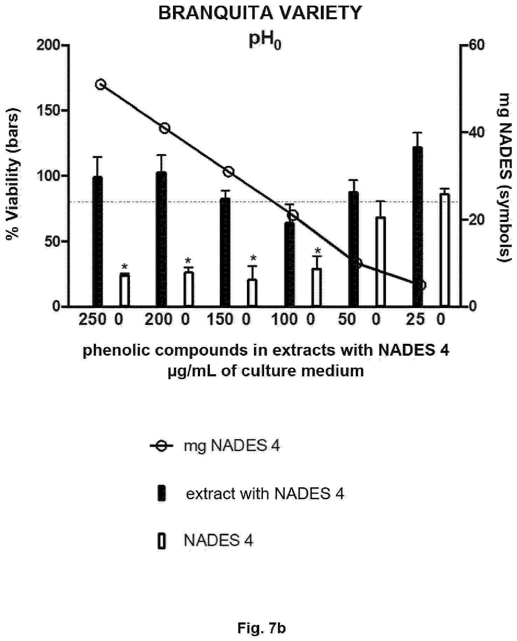

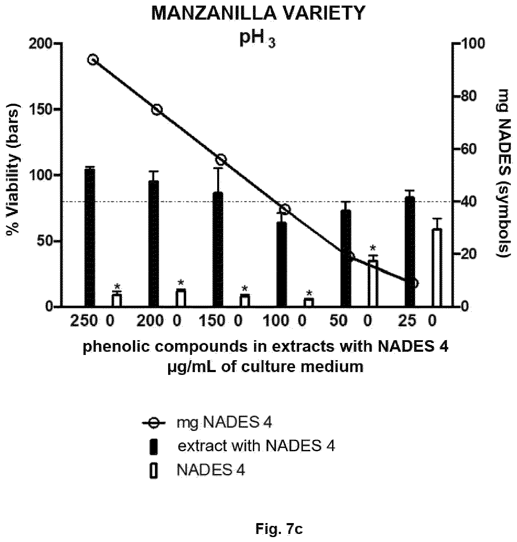

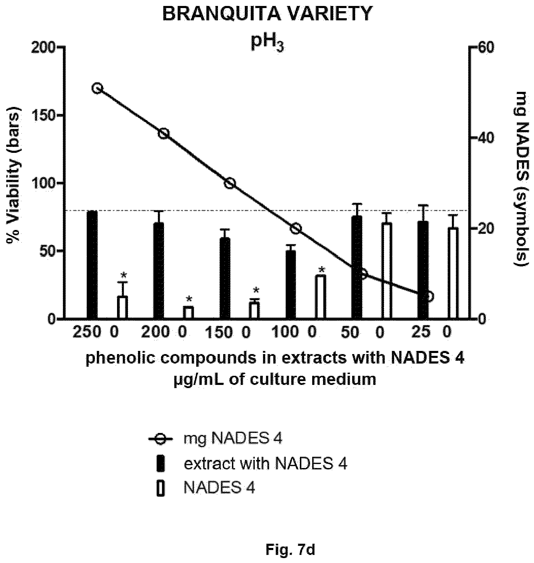

**Fig. 7.** Viability of BV2 microglia cells treated with different concentrations of NADES 4 with or without extract in the Manzanilla and Branquita varieties at pH 0 and 3, (**7a**) Manzanilla pH 0, (7b) Branquita pH 0, (**7c**) Manzanilla pH 3, (**7d**) Branquita pH 3. The concentration of phenolic compounds in the extracts ($\mu$g/ml) is shown on the X-axis and the mg of NADES 4 for each sample is shown on the right Y-axis. The results are expressed as a percentage (%) of live cells, according to the following relationship: % = O.D of the treated cells x 100/O.D of the control cells. P<0.5. * Significantly different for NADES 4 versus NADES 4 with its extract. The dotted line indicates the value below which cellular toxicity occurs.

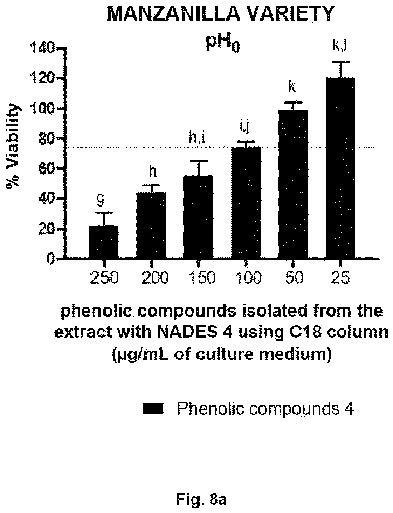

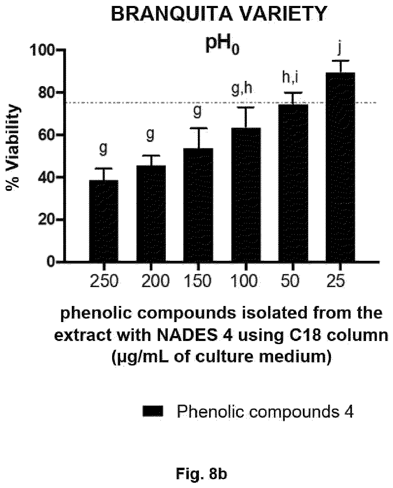

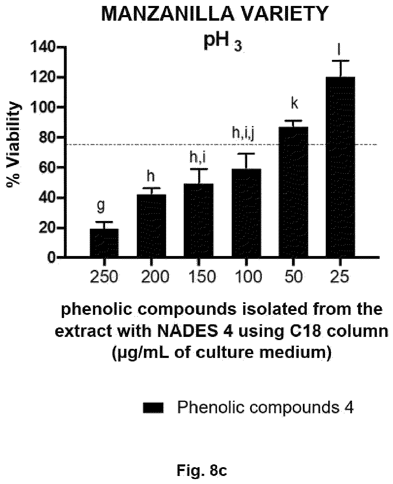

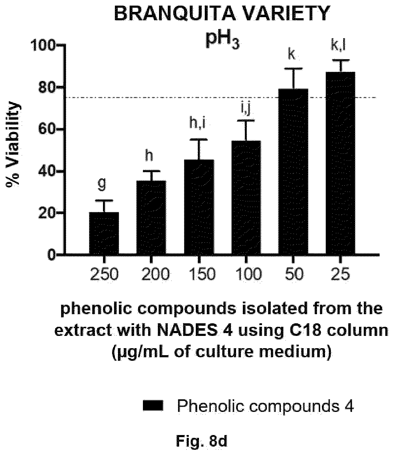

**Fig. 8.** Viability of BV2 microglia cells treated with different concentrations of phenolic compounds isolated from the extracts with NADES 4 using a C18 column in Manzanilla and Branquita varieties at pH 0 and 3, (**8a**) Manzanilla pH 0, (**8b**) Branquita pH 0, (**8c**) Manzanilla pH 3, (**8d**) Branquita pH 3. The results will be expressed as a percentage (%) of live cells, according to the following relationship: % = O.D of the treated cells x 100/O.D of the control cells. P<0.5. Different letter for each column denotes significantly different for the different concentrations of phenolic compounds (g, h, I, j, k) The dotted line indicates the value below which cellular toxicity occurs.

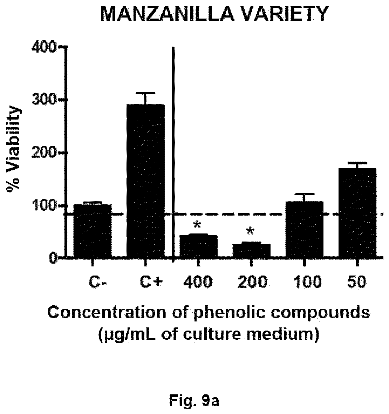

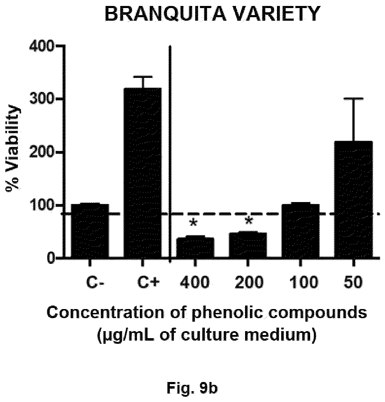

**Fig. 9.** Viability of BV2 microglia cells treated with phenolic compounds extracted using C-18 columns from extracts with NADES 4 after their digestion with gastrointestinal enzymes (digestates). BV2 microglia cells were incubated in culture medium in the absence (C-) and presence of serum (C+) and treated with different concentrations of digestates at concentrations of 400, 200, 100 and 50 $\mu$g/ml of culture medium in the Manzanilla (**9a**) and Branquita (**9b**) varieties. The cytotoxic effect is shown below 80% viability percentage (horizontal dashed line), *(p < 0.01), each of the samples versus C-. n=6.

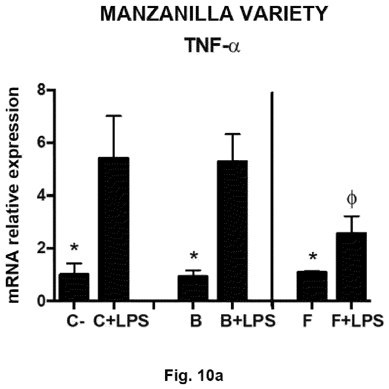

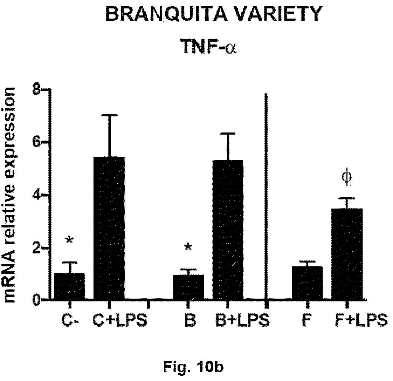

**Fig. 10.** Expression of tumour necrosis factor alpha (TNF-$\alpha$) in murine microglia cells stimulated with digestates from extracts with NADES 4 from the Manzanilla (**10a**) and Branquita (**10b**) varieties. Murine microglia cells were incubated in culture medium in the absence (C-) and presence of LPS (inducing lipopolysaccharide) (C+LPS), and stimulated with targets (B) and with phenolic compounds of the digestates (F), in both cases, in the absence and presence of LPS. On the X-axis, the relative expression of TNF-$\alpha$ is shown. *(p < 0.01), C- versus C+LPS; B versus B+LPS, F versus F+LPS; Ø (p < 0.01), B+LPS versus F+LPS, n=6.

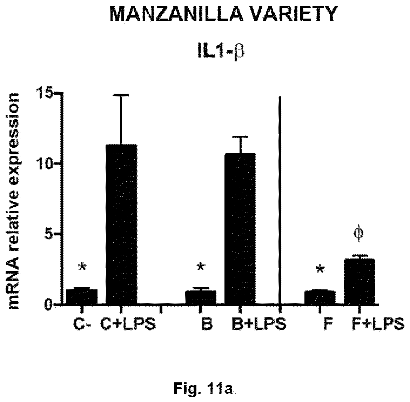

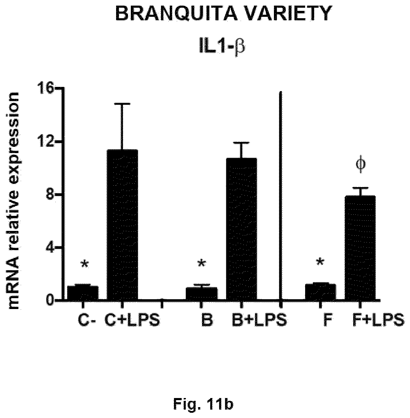

**Fig 11.** Expression of the interleukin 1-beta factor (IL-1$\beta$) in murine microglia cells stimulated with digestates from extracts with NADES 4 from the Manzanilla (**11a**) and Branquita (**11b**) varieties. Murine microglia cells were incubated in culture medium in the absence (C-) and presence of LPS (C+LPS), and stimulated with targets (B) and with phenolic compounds of the digestates (F), in both cases, in the absence and presence of LPS. On the X-axis, the relative expression of IL-1β is shown. *(p < 0.01), C- versus C+LPS; B versus B+LPS, F versus F+LPS; Ø (p < 0.01), B+LPS versus F+LPS, n=6.

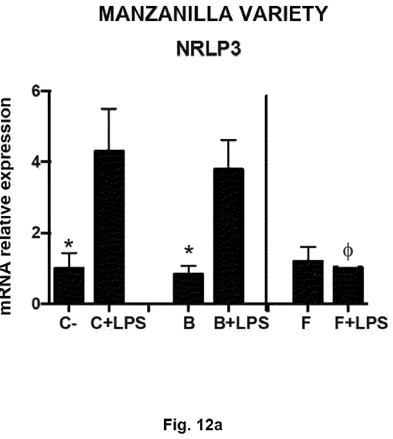

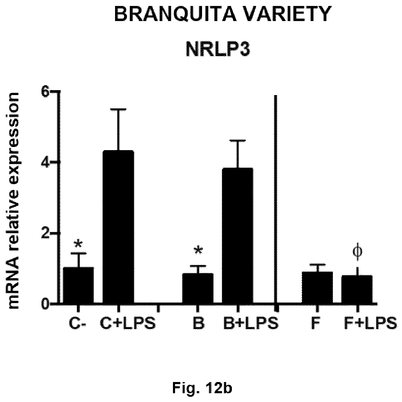

**Fig 12.** Expression of NLRP3 inflammasome in murine microglia cells stimulated with digestates from extracts with NADES 4 from the Manzanilla (**12a**) and Branquita (**12b**) varieties. Murine microglia cells were incubated in culture medium in the absence (C-) and presence of LPS (C+LPS), and stimulated with targets (B) and with phenolic compounds of the digestates (F), in both cases, in the absence and presence of LPS. On the X-axis, the relative expression of IL-1β is shown. *(p < 0.01), C- versus C+LPS; B versus B+LPS; Ø (p < 0.01), B+LPS versus F+LPS, n=6.

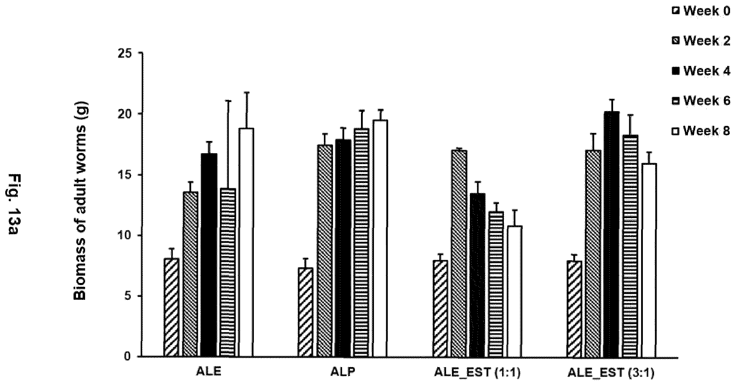

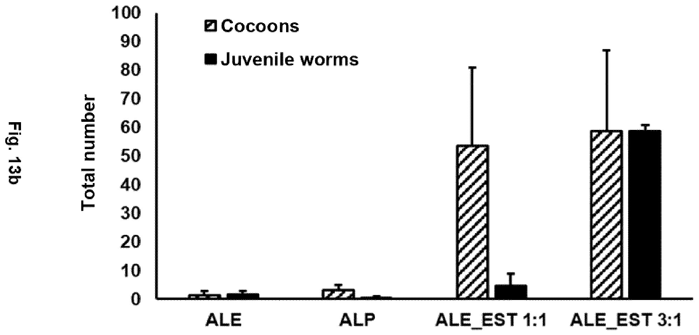

**Fig. 13.** Vermicompost of alpeorujo extracted with NADES 4. (**13a**) Evolution of the total biomass (g) of adult worms in the different treatments. ALE = alpeorujo extracted with NADES 4, ALP = fresh alpeorujo; EST = horse manure; ALE_EST= mixture of alpeorujo and manure. (**13b**) total number of cocoons and juvenile worms produced in each treatment throughout the assay (n=8 weeks). ALE = alpeorujo extracted with NADES 4, ALP = fresh alpeorujo; EST = horse manure; ALE_EST= mixture of alpeorujo and manure. (P<0.05).

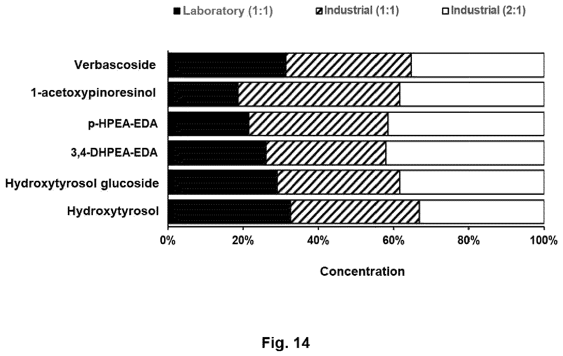

**Figure 14.** Concentration of individual phenolic compounds expressed as a percentage that are extracted from fresh alpeorujo using NADES 4 on a laboratory scale using the alpeorujo:solvent ratio (1:1) and on an industrial scale using the alpeorujo:solvent ratios (1:1) and (2:1). Analyses were performed on UHPLC-UV-MS. (n=2).

**Figure 15.** Summary diagram of the fresh alpeorujo recovery processes based on circular economy principles, from the production of virgin olive oil (VOO) to the production of fertiliser or organic soil conditioner. Process 0, extraction of VOO and obtaining product 0, which is VOO and its by-product 0, fresh alpeorujo. Process 1, extraction of phenolic compounds from fresh alpeorujo using NADES 4, obtaining product 1, which is the phenolic extract with nutraceutical or antimicrobial uses, and by-product 1, alpeorujo extracted with NADES 4 (ALE). Process 2, vermicomposting of ALE, obtaining only product, the organic soil conditioner or fertiliser that returns to the VOO production cycle, closing the cycle with essentially zero residue.

# EXAMPLES

Next, the invention will be illustrated by means of assays carried out by the inventors which demonstrate the effectiveness of the product of the invention.

## Example 1: Preparation of NADES

The components of NADES, malic acid and sucrose in a 1:1 w/w ratio, with 8-35% by weight of water, are mixed. The corresponding components are mixed and kept under continuous stirring at a temperature of 50-80°C until the solvent is formed as a transparent and stable gel.

## Example 2: Phenolic extraction method:

Alpeorujo and NADES are weighed in an alpeorujo: NADES ratio between 5:1 and 1:5 w/w, stirring for 10 min to 6 h, at a temperature of 20-90°C. Next, the mixture is centrifuged between 1000 rpm and 10,000 rpm to separate the phase consisting of phenolic extract plus NADES 4 from the partially extracted alpeorujo. Two extractions can be performed by taking the phenolic extract phase and the extraction process is repeated on the partially extracted alpeorujo phase. The 2 extract phases resulting from the 2 extractions are combined and constitute the "phenolic extract". The alpeorujo phase after 1 extraction or after 2 extractions with NADES is called "alpeorujo extracted with NADES".
Through this method, liquid extracts (or gels) rich in phenols combined with NADES are obtained, which composition allows their use as nutraceuticals or as food additives given that said liquid extracts are non-toxic for said use and for phytosanitary use as antimicrobials, since NADES has been described for that use.
Moreover, alpeorujo extracted with NADES 4 (ALE) is obtained through this method, in other words, with a low concentration of phenols, useful for use in vermicompost when combined with organic substrates.
The method of the invention is summarised in figure 15.

## Example 3: Extractive efficiency of the acidic NADES understudy

This assay determined the extracting capacity of two acid-based natural deep eutectic solvents (NADES) and it was compared with NADES of different formulations and with conventional solvents. To that end, the phenolic content extracted by 16 different solvents was analysed on the phenolic compounds of fresh alpeorujo of the Picual variety (**Figs. 2, 3**).

**Table 1:** Composition and pH of the different solvents used.

| Solvent | Components | | | Molar Ratio | % Water | pH |
|---|---|---|---|---|---|---|
| | 1 | 2 | 3 | | | |
| 1 | lactic acid | betaine | - | 2:1 | - | 0.28 |
| 2 | lactic acid | D-(-)-fructose | - | 5:1 | - | 0.37 |
| 3 | glycolic acid | D-(-)-fructose | - | 1:1 | 13 | 0.33 |
| 4 | malic acid | sucrose | - | 1:1 | 19 | 0.71 |
| 5 | citric acid | xylitol | - | 1:1 | 17 | 0.3 |
| 6 | citric acid | sorbitol | - | 1:1 | 19 | 0.34 |
| 7 | choline chloride | xylitol | - | 2:1 | 11 | 3.33 |
| 8 | choline chloride | glycerol | - | 1:2 | - | 6.47 |
| 9 | methanol : water mixture (80%) | | | | | |
| 10 | methanol : water mixture (50%) | | | | | |
| 11 | betaine | sucrose | - | 2:1 | 13 | 6.48 |
| 12 | choline chloride | glycolic acid | oxalic acid | 1:1.7:0.3 | - | 0.15 |
| 13 | D-(+)-glucose | glycerol | - | 1:1 | 21 | 1.6 |
| 14 | Distilled water | | | | | |
| 15 | D-(+)-fructose | D-(+)-glucose | sucrose | 1:1:1 | 22 | 6.05 |
| 16 | D-(+)-fructose | sucrose | - | 1:1 | 18 | 6.2 |

Table 1 shows the different components of the solvents used for extracting alpeorujo polyphenols. The NADES 4 solvent is the acidic formulation that is the subject of this study. Solvents 9 and 10 correspond to aqueous mixtures of the solvent methanol and solvent 14 corresponds to water; these solvents were used as extraction controls since they are the usual conventional mixtures for extracting polyphenols.

Figure 2 shows the individual phenol concentration obtained by each solvent in descending order. Solvents 1, 2 and 4 exhibited a similar extraction pattern of phenolic compounds and the highest extractive efficiencies (see figures 2 and 3). NADES 4 exceeded 3900 mg/kg in wet weight of extracted polyphenols in the Picual variety, quantities statistically similar to those of the remaining more extractive acid-based solvents used.

The NADES 4 solvent was significantly more extractive than the conventional aqueous methanol mixtures (9, 10) and water (14) (see table 1).

Phenolic extracts were obtained using NADES 4 from fresh alpeorujo of the Manzanilla and Branquita varieties to determine their bioactive effect. Figures 4 and 5 show the concentration of 14 phenolic and oleosidic compounds that are characteristic of olives and their fresh derivatives such as alpeorujo, quantified individually.

**Example 4: Determination of the toxicity of acid NADES in a study on phytopathogenic bacteria and the antimicrobial activity of phenolic extracts obtained by NADES 4 from fresh alpeorujo.**

The *in vitro* toxicity of acid-based NADES 4 (malic acid/sucrose) on phytopathogenic bacteria of agronomic interest was analysed. The antimicrobial activity of the phenolic extracts obtained with NADES 4 from fresh alpeorujo of the Picual, Manzanilla and Branquita varieties was determined.

Toxicity and antimicrobial activity analyses were carried out with strains obtained from the Spanish Type Culture Collection (CECT). *Erwinia amylovora* CECT 222, *Erwinia toletana* CECT 5263, *Xanthomonas campestris* CECT 97, *Pseudomonas syringae* CECT 4429, *Pseudomonas savastanoi* CECT 5019 and, *Rhizobium radiobacter* CECT 4119.

For each experiment, the strain was incubated under aerobic conditions with stirring at 29°C for 24 h, resulting in broths with approximate values of $10^9$ CFU/ml. The antimicrobial capacity of the extracts was determined by calculating the minimum inhibitory capacity (MIC) by serial dilution of the extracts in 96-well microtitre plates. The corresponding dilutions were carried out to obtain $10^6$ CFU/ml per well.

For the toxicity analysis of NADES 4, the effects of NADES 4 were compared at initial acidic pH, which is the original pH of NADES (pH 0.71), and adjusted to pH 5.5 - 6 with $NaHCO_3$. The pH was adjusted to avoid an excessively acidic medium incompatible for optimal bacterial development. Other conventional solvents were also included as a control, such as low-toxicity glycerol used for the cryopreservation of bacteria and dimethyl sulfoxide (DMSO) also considered to have low toxicity in different cell types. It is considered that a solvent exhibits low toxicity when it does not inhibit the growth of microorganisms at a concentration in the culture medium equal to or greater than 10%. Ethanol, which is a solvent toxic to bacteria, was also used. These 3 solvents make it possible to establish comparisons with the NADES subject of this study. The obtained results are shown in figure 6.

In all cases, it was observed that NADES 4 adjusted to pH 5.5 - 6 exhibited low toxicity against all the bacterial strains analysed (Fig. 6a). By adjusting the pH of NADES, the minimum inhibitory concentration of NADES increased two-fold in all cases except for the strain of *X. campestris* in which the inhibition by NADES 4 was not affected by pH. NADES 4 is therefore a low-toxicity solvent for the bacteria under study even at the original acidic pH.

Next, the effect on the bacterial growth of NADES 4 at pH adjusted to 5.5-6 and three conventional solvents was studied, with glycerol and dimethyl sulfoxide (DMSO) as positive controls due to their low toxicity and ethanol as a negative control due to its powerful toxicity against bacteria (figure 6b).

NADES 4 at adjusted pH exhibited a lower or equal percentage of inhibition than all the solvents analysed. Thus, it was less inhibitory than even the positive controls for the strains of the genus *Pseudomonas,* for *X. campestris* and *R. radiobacter* and had an identical percentage of inhibition as glycerol for strains of the genus *Erwinia.* NADES 4 was between 4 and 125 times less toxic than the negative control, ethanol, and 4 times less toxic than DMSO.

These results indicate its possible use as a cryoprotectant or carrier of substances of interest for working with bacteria without inhibiting their growth.

The antimicrobial activity of the extracts obtained by NADES 4 from the alpeorujo of three varieties of olives, Manzanilla, Picual and Branquita, against the 6 phytopathogenic strains was determined. The pH of the extracts was adjusted to optimal values for bacterial growth at pH 5.5 - 6 with NaHCOs.

**Table 2.** Bactericidal effect (MIC) mg/l of the phenolic extracts obtained with NADES 4 from the Manzanilla, Picual and Branquita olive varieties on the strains *Erwinia amylovora* CECT 222, *Erwinia toletana* CECT 5263, *Xanthomonas campestris* CECT 97, *Pseudomonas syringae* CECT 4429, *Pseudomonas savastanoi* CECT 5019 and, *Rhizobium radiobacter* CECT 4119.

| Extract with NADES 4 | MIC (mg/L) | | |
|---|---|---|---|
| | **Manzanilla** | **Picual** | **Branquita** |
| *Erwinia amylovora* | 503 | 740 | 1190 |
| *Erwinia toletana* | 1007 | 740 | 1190 |
| *Xanthomonas campestris* | 252 | 185 | 149 |
| *Pseudomonas syringae* | 252 | 370 | 595 |
| *Pseudomonas savastanoi* | 252 | 370 | 297 |
| *Rhizobium radiobacter* | 503 | 370 | 1190 |
| $N_0 = 10^6$ CFU/ml, n=3. | | | |

**Table 2** shows how the extract obtained with NADES 4 from fresh alpeorujo of three different varieties exhibits antimicrobial activity against the 6 bacterial strains under study.

All phenolic extracts obtained with NADES 4 exhibited antimicrobial effect. The extracts of the Manzanilla and Picual varieties were more inhibitory to bacterial growth than those of Branquita for all strains except for X. *campestris* with the lowest MIC value (149 mg/ml). However, these extracts contain a lower total phenolic concentration than those of the Branquita variety, but they are richer in seco-iridoid derivatives and simple phenols with known antimicrobial effects (Brenes, M., Garcia, A., de los Santos, B., Medina, E., Romero, C. & de Castro, A., Romero, F. (2010). Olive glutaraldehyde-like compounds against plant pathogenic bacteria and fungi. Food Chemistry, Vol. 125, 1262-1266.). Bacteria of the genus *Erwinia* were the most resistant to the phenolic extracts of the 3 varieties, the extracts of Picual on E. *toletana* exercising the greatest antimicrobial effect, and the genus *Pseudomonas* was the most sensitive to the extracts of the 3 varieties.

These results are related to the bacterial growth inhibitory capacity of the different phenols that make up the extract. The antimicrobial effect of phenolic compounds is well known, especially from oleuropein derivatives such as 3,4-DHPEA-EDA (oleacein), other seco-iridoids, oleosides, alcohols such as hydroxytyrosol, its glycosides and to a lesser extent phenylpropanoids such as verbascoside.

These results indicate that the phenolic extracts obtained with NADES 4 from the Manzanilla, Picual and Branquita varieties exhibit antimicrobial effect on all the strains studied, which is comparable to that exhibited by other phenolic extracts of natural origin.

The antimicrobial effect of the extracts obtained with NADES 4 would be related to the phenolic compounds extracted by NADES 4 from fresh alpeorujo and not with NADES 4 itself, since the latter showed low toxicity on the microorganisms analysed (**fig. 6**).

**Example 5: Cytotoxicity of the phenolic extracts obtained with NADES 4 in murine BV2 microglia cells.**

The *in vitro* cytotoxicity of acid-based NADES 4 (malic acid/sucrose) of fresh alpeorujo from the Manzanilla and Branquita varieties in murine BV2 microglia cells was analysed in order to determine its potential as a nutraceutical against neuronal diseases such as Alzheimer's due to its anti-inflammatory and antioxidant properties.

Figure 7 shows the percentage of live cells after incubation with NADES 4 (0 phenolic compounds) and the fresh alpeorujo extracts from the Manzanilla and Branquita varieties with NADES 4 with different concentrations of phenolic compounds (250, 200, 150, 100, 50 and 25 $\mu$g/ml of cell medium) at pH 0 and 3. The results showed that NADES 4 are cytotoxic for microglia cells starting from a concentration greater than 50-75 mg of NADES per millilitre of medium, both at pH 0 and pH 3. Surprisingly, the cytotoxic effect of NADES was counteracted by alpeorujo extracts in both varieties and both pHs. Cell viability was higher for the Manzanilla variety than for Branquita.

The phenolic compounds present in the extracts obtained with NADES 4 from the Manzanilla and Branquita varieties were isolated using a solid phase column with C18 packing and their cytotoxic effect on murine microglia cells was determined.

Figure 8 shows the *in vitro* cytotoxic effect in murine microglia cells of the phenolic compounds isolated from the extracts obtained with NADES 4 from the Manzanilla and Branquita varieties. The phenolic compounds were washed using a solid phase column with C18 packing after cell incubation. The phenolic compounds of the Manzanilla variety exhibited a cytotoxic effect at high concentrations (250-150 $\mu$g/ml) in microglia cells at both pH 0 and pH 3. The Branquita variety exhibited cytotoxicity for concentrations of phenolic compounds of 250-100 $\mu$g/ml at both pHs.

Taking into account this *in vitro* data, the extracts isolated with NADES 4 could be used at initial pH, which would avoid producing modifications of the phenolic compounds due to changes in pH. Both extracts seem to contain compounds other than phenols that promote cell viability, counteracting the cytotoxic effect that phenols have in isolation at high concentrations in these cells. This data suggests that the extracts of the NADES studied could allow the use of high concentrations of phenolic compounds, avoiding their cytotoxic factor in microglia cells and being able to exert anti-inflammatory and antioxidant functions that are typical of these compounds.

**Example 6: Anti-inflammatory effect of the phenolic extracts obtained with NADES 4 in murine BV2 microglia cells.**

Reactive glial cells release a series of factors involved in the inflammatory response, the most important being a group of cytokines with a marked pro-inflammatory character, such as tumour necrosis factor alpha (TNF-$\alpha$) and interleukin 1-beta (IL-1$\beta$). Cytokines of the IL-1b family also require an activation process controlled by several complexes called inflammasomes, among which the NLRP3 inflammasome is an important complex in the activation of BV2 cells.

The anti-inflammatory effect of the extracts obtained with NADES 4 in murine microglia cells was studied.

Prior to incubation with microglia cells, the extracts were subjected to *in vitro* enzymatic digestion, (Jaramillo, S., Muriana F.J.G. Muriana, Guillen, R., Jimenez-Araujo, A., Rodriguez-Arcos, R., Lopez, S. (2016). Saponins from edible spears of wild asparagus inhibit AKT, 2 p70S6K, and ERK signalling, and induce apoptosis through 3 G0/G1 cell cycle arrest in human colon cancer HCT-116 cells. Journal of Functional Foods 26, 1-10), in order to simulate the changes produced by the samples during the biological process of digestion. Finally, the phenolic compounds were extracted from the digested extracts using a C18 column before incubation with microglia cells.

Subsequently, the maximum concentration at which said compounds are not cytotoxic to cells was determined, establishing this value at 100 $\mu$g/ml (**fig. 9**).

The cells were cultured in culture medium in the presence and absence of LPS (100 ng/ml). In parallel experiments, the cells were incubated with the purified digestates at a non-cytotoxic concentration of 100 $\mu$g/ml, in the presence and absence of LPS. The expression of TNF-$\alpha$ (**fig. 10**), IL-1$\beta$ (**fig. 11**) and NRLP3 (**fig. 12**), were measured by quantitative PCR.

The results showed that the expression of TNF-$\alpha$, IL-1$\beta$ and NRLP3 induced by LPS increased significantly in relation to the controls and targets (p < 0.01), but the increase was significantly lower when the cells were stimulated with the purified phenolic compounds in both varieties. The effect was greater in the extracts obtained in the Manzanilla variety except in NRLP3, which were the same.

The study of TNF-$\alpha$, IL-1$\beta$ and NRLP3 expression reflects a marked anti-inflammatory character of the purified phenolic compounds obtained from the extracts with NADES 4 in the Manzanilla variety and to a lesser extent in the extracts obtained with the Branquita variety.

**Example 7: Vermicompost of alpeorujo extracted with NADES 4**

Alpeorujo extracted with NADES 4 (ALE) exhibits low palatability for worms of the species *Eisenia fetida* used in this study, since its low porosity and low pH (2.5) limit this product from vermicomposting. To obtain good results with the vermicomposting of alpeorujo extracted with NADES 4, three processes must be carried out. The first of them consists of pre-composting the alpeorujo extracted with NADES 4 for at least 8 weeks to facilitate the degradation of organic matter and the mineralisation of nitrogen compounds. Furthermore, during this process, the pH of ALE rises to optimal values for the life of the worms. In a second process, ALE must be mixed with materials that improve its physical-chemical structure and its palatability for worms. The final process consists of using a percentage of manure in each assay mixture to be vermicomposted, which is a very palatable substrate for worms, in proportion from 20 to 50% (w/w) of material to be composted, with the aim of facilitating the acceptance of the diet by the worms. Each of these assays was carried out in containers that we call pots.

Once these three processes have been verified, 4 treatments were established: extracted alpeorujo (ALE) and two mixtures of ALE with horse manure (EST) in the proportions indicated below in (w/w), ALE-EST (1:1) and ALE-EST (3:1). Furthermore, a treatment only with fresh unextracted alpeorujo (ALP) was added. The physical-chemical characteristics of the treatments at the beginning of vermicomposting are presented in **table 3.**

**Table 3:** Physico-chemical properties of the treatments at the beginning of the assay before starting the vermicomposting process of each substrate and ratio (w/w) of each component in the mixtures. Extracted alpeorujo (ALE), fresh alpeorujo (ALP), horse

| Determination | Treatment | | | | |
|---|---|---|---|---|---|
| | **ALE** | **ALE_EST (1:1)** | **ALE_EST (3:1)** | **ALP** | **EST** |
| **Humidity (%)** | 45.09±1.29 | 66.05±0.35 | 61.94±0.01 | 51.90±0.18 | 54.67±0.02 |
| **pH** | 2.45±0.01 | 8.50±0.29 | 4.29±0.03 | 6.35±0.02 | 8.17±0.07 |
| **EC (mS/cm2)** | 1.92±0.04 | 1.33±0.11 | 3.94±0.35 | 2.58±0.44 | 3.28±0.18 |
| **TOM (%)** | 97.50±0.23 | 54.11±2.34 | 71.43±2.12 | 92.24±0.23 | 30.20±0.85 |
| **C (%)** | 51.65±1.52 | 31.17±2.42 | 37.87±0.52 | 51.31±0.08 | 18.89±0.62 |
| **N (%)** | 0.33±0.06 | 2.02±0.27 | 1.41±0.06 | 0.83±0.02 | 1.55±0.07 |
| **C/N** | 161±27.19 | 16±3.33 | 27±1.60 | 62±1.78 | 12±0.13 |
| **P (%)** | 0.01±0.0 | 0.01±0.0 | 0.13±0.01 | 0.07±0.0 | 0.04±0.0 |
| **K (%)** | 0.26±0.04 | 0.96±0.04 | 0.72±0.03 | 1.5±0.10 | 1.66±0.23 |
| **Ca (%)** | 0.03±0.0 | 4.59±0.22 | 3.21±0.13 | 0.13±0.07 | 4.78±0.63 |
| **Mg (%)** | 0.02±0.0 | 0.23±0.0 | 0.17±0.0 | 0.09±0.0 | 0.27±0.03 |
| **Na (%)** | nd | 0.12±0.0 | 0.08±0.0 | 0.01±0.0 | 0.25±0.0 |
| **Fe (mg/kg)** | 56.10±5.38 | 67.70±4.43 | 382.84±30.44 | 36.68±0.35 | 65.81±3.94 |
| **Mn (mg/kg)** | 2.14±0.02 | 2.39±0.19 | 3.70±0.44 | 7.78±0.67 | 2.40±0.04 |
| **Cu (mg/kg)** | 0.65±0.06 | 4.69±0.44 | 3.35±0.29 | 5.37±0.37 | 12.49±1.22 |
| **Zn (mg/kg)** | 1.76±0.12 | 38.89±3.12 | 39.27±4.81 | 8.19±0.56 | 33.77±1.74 |
| manure (EST). The concentration of each element is expressed as a percentage and in mg/kg of sample. EC, electric conductivity, TOM total organic matter. (n= 4). | | | | | |

After pre-composting and preparation of the base material mixtures described, the vermicomposting process began with 30 worms of the species *Eisenia fetida* per pot. In total, 4 pots were established for each treatment, which remained for 8 weeks under optimal conditions of humidity and temperature. The main results of the vermicomposting process of alpeorujo extracted with NADES 4 are summarised below:

ALE treatment can be vermicomposted, but like ALP it has low palatability for worms. Its transformation takes between 3-5 weeks compared to treatments with manure, which are more palatable to worms (**Fig. 13a**). Consequently, the total number of cocoons and juvenile worms in the ALE and ALP treatments was significantly lower than in the treatments with manure ALE_EST (1:1) and ALE_EST (3:1) (**Fig. 13b**).

The addition of manure to ALE not only improved the initial pH of ALE, but it allowed the worms to transform the ALE in just 4-5 weeks. Both treatments with manure reached a similar total biomass of adult worms (**Fig. 13a**).

The total number of cocoons was also similar (**Fig. 13b**). Nevertheless, the number of juvenile worms was lower in the treatment ALE_EST (1:1) possibly due to the lack of food for the young worms (**Fig. 13b**). The treatment ALE_EST (1:1) was the one with the highest palatability for worms. The 30 adult worms ate all the substrate of the treatment ALE_EST (1:1) in just 4 weeks, leaving hardly any food for the juvenile worms. To avoid this problem and even if the vermicomposting process takes a little longer, it is recommended to reduce the number of worms (maximum 15-20 worms) for the same volume of substrate or add more substrate from the treatment ALE_EST (1:1) so that the juvenile worms can develop properly. Compared to the treatment ALE_EST (1:1), the treatment ALE_EST (3:1) behaved in a very similar way and also allows transforming ALE into vermicompost 3 more times, without exhibiting problems for worm growth and reproduction.

The results obtained indicate that ALE is a by-product that can be transformed into vermicompost by previously carrying out precomposting that facilitates the increase of pH to values compatible with the life of the worms (pH$\geq$5). The treatment ALE_EST (3:1) seems to be the most viable and cost-effective option to transform ALE into vermicompost.

**Example 8: Stability test of the phenolic extracts of NADES 4.**

The stability of the phenolic extracts obtained with NADES 4 from the 3 varieties of fresh alpeorujo was determined after a period of 12 months of storage in darkness at room temperature. The assay was carried out by preserving 5 ml of each extract in closed vials. After storage, the presence or absence of precipitation was determined. As a result, no precipitation was detected in any of the extracts and it was concluded that they were stable under the assay conditions.

**Example 9: Industrial scaling.**

The phenolic extract and extracted alpeorujo (ALE) were obtained from fresh alpeorujo extracted with NADES 4 on an industrial scale. The process was carried out in the facilities at the Seville Fat Institute (*Institute del Grasa de Sevilla*) (experimental oil mill); fresh alpeorujo preserved frozen at -20°C was used from its production to its use.

75 kilograms of NADES 4 were prepared by mixing the components in an industrial stirrer at 60°C until the solvent was formed (30-40 minutes).

The extracts were carried out with two ratios of alpeorujo and different solvents:

- ratio (1:1), 25 kilograms of fresh alpeorujo plus 25 kilograms of NADES 4, and

- ratio (2:1), 100 kilograms of fresh alpeorujo plus 50 kilograms of NADES 4.

The extractions were carried out in industrial equipment at the aforementioned experimental oil mill; the equipment used is usually used for the industrial extraction of virgin olive oil.

NADES 4 was added to fresh alpeorujo in a ratio (1:1) or (2:1) and the mixture was stirred for 1 hour in an industrial mixer at 40°C, talc was added as a technological adjuvant during mixing to improve the separation of solid and extract phases. Next, the phenolic extract phase was separated with the NADES 4 solvent from the extracted alpeorujo (ALE) by centrifugation in a horizontal centrifugal separator or decanter (centrifuge for obtaining olive oil in an oil mill), the phenolic extract was sieved and centrifuged again for clarification.

Phenolic extracts from fresh alpeorujo with NADES 4 were obtained using the alpeorujo:solvent ratios (1:1) and (2:1). In order to know the extractive efficiency of phenols of the method on an industrial scale, the phenolic compounds of the extracts thus obtained were analysed and their phenolic concentrations were compared with that of extracts obtained in the laboratory from the same alpeorujo (using an ABENCOR brand extraction system) with the alpeorujo:solvent ratio (1:1) and with similar production conditions of time, temperature and centrifugation speed.

The phenolic concentration expressed as a percentage of the industrial extracts obtained with NADES 4 in the ratios (1:1) and (2:1) and on a laboratory scale in the ratio (1:1) is shown in **figure 14**.

When comparing the industrial extracts in a ratio (1:1) with those obtained in the laboratory in the same ratio, the concentrations in phenolic compounds did not show significant differences except for the compounds 3,4-DHPEA-EDA, *p*-HPEA-EDA and 1-acetoxypinoresinol in which the industrial method was more extractive. When the two industrial methods in ratios (1:1) and (2:1) were compared, the ratio (2:1) was significantly more extractive for the hydroxytyrosol glucoside, 3,4-DHPEA-EDA, *p*-HPEA-EDA and 1-acetoxypinoresinol compounds. These results suggest that the alpeorujo:solvent ratio (2:1) would be more effective for the industrial extraction of phenolic compounds from alpeorujo with NADES 4.

**Claims**

1. A method for obtaining a phenolic extract from alpeorujo that comprises the following steps:

   i. preparing the natural deep eutectic solvent (NADES 4) selected from malic acid and sucrose in a 1:1 w/w ratio, containing between 8% and 35% by weight of water; and
   ii. adding NADES 4 from step (i) to fresh alpeorujo in an alpeorujo: NADES ratio between 5:1 and 1:5 w/w, stirring for a time of between 10 minutes and 6 hours, at a temperature between 20°C and 90°C, and centrifuging between 1000 rpm and 10,000 rpm to separate the phenolic extract phase with the solvents of the alpeorujo extracted (ALE) with NADES 4.

2. The method according to claim 1, wherein step (ii) is repeated one or more times.

3. A phenolic extract from alpeorujo obtained by the method described in any of claims 1 to 2.

4. Use of the phenolic extract according to claim 3, as a nutraceutical, food additive or phytosanitary product.

5. The use according to claim 4, as a phytosanitary product in the treatment of microbial plant diseases, preferably for the treatment of microbial plant diseases caused by bacteria of the genera *Erwinia, Pseudomonas, Xanthomonas* and *Rhizobium*

6. An extracted alpeorujo (ALE) obtained by the method described in any of claims 1 to 2.

7. Use of the extracted alpeorujo (ALE) according to claim 6, as an organic supplement for compost and vermicompost.

8. The use of the extracted alpeorujo (ALE) according to claim 7, as an organic supplement for vermicompost, **characterised in that** ALE treatment is carried out with worms of the species *Eisenia fetida.*

**Fig. 1**

Fig. 2

**Fig. 3**

Fig. 4

Fig. 5

**Fig. 6**

Fig. 7a

Fig. 7b

**Fig. 7c**

Fig. 7d

**MANZANILLA VARIETY**

pH$_0$

Fig. 8a

## BRANQUITA VARIETY
### $pH_0$

phenolic compounds isolated from the
extract with NADES 4 using C18 column
(µg/mL of culture medium)

■ Phenolic compounds 4

**Fig. 8b**

**MANZANILLA VARIETY**
**pH 3**

Fig. 8c

Fig. 8d

Fig. 9a

BRANQUITA VARIETY

Concentration of phenolic compounds
(µg/mL of culture medium)

Fig. 9b

## MANZANILLA VARIETY
## TNF-α

Fig. 10a

BRANQUITA VARIETY

TNF-α

Fig. 10b

**MANZANILLA VARIETY**

**IL1-β**

Fig. 11a

# BRANQUITA VARIETY

## IL1-$\beta$

Fig. 11b

Fig. 12a

# BRANQUITA VARIETY

## NRLP3

Fig. 12b

Fig. 13a

**Fig. 13b**

Fig. 14

Olives

PROCESS 0

Product 2 = Fertiliser

VOO = Product 0

PROCESS 2

Worm

FRESH
ALPEORUJO = By-product 0

NADES

By-product 1 = ALPEORUJO
EXTRACTED
WITH NADES
(ALE)

PROCESS 1

Phenolic extract = Product 1

Nutraceutical, antimicrobial

Fig. 15

# INTERNATIONAL SEARCH REPORT

| | International application No. |
|---|---|
| | PCT/ES2022/070561 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)
B01D, A23L, A01N, C05F, A01K

Documentation searched other than minimum documentation to the extent that such documents are included in the fields searched

Electronic data base consulted during the international search (name of data base and, where practicable, search terms used)

EPODOC, INVENES, WPI, CAPLUS, BIOSIS, MEDLINE, EMBASE, BD-TCXTE, INTERNET

**C. DOCUMENTS CONSIDERED TO BE RELEVANT**

| Category* | Citation of document, with indication, where appropriate, of the relevant passages | Relevant to claim No. |
|---|---|---|
| P, A | WO 2021180996 A1 (C.S.I.C. and Universidad de Sevilla) 16/09/2021, Claims. | 1-8 |
| P, A | MORGANA N M et al. NADES for food industry innovation: novel bioadditives based on olive oil byproducts. Food and Bioproducts Processing 14/05/2022, vol. 134, pages 193 - 201, ISSN 0960-3085 (print), DOI: 10.1016/j.fbp.2022.05.007 | 1-8 |
| A | FERNÁNDEZ M. et al. Novel approaches mediated by tailor-made Green solvents for the extraction of phenolic compounds from agro-food industrial by-products. Food Chemistry, 2018, vol. 239, pages 671 - 678, ISSN 1873-7072 (Electronic), DOI: 10.1016/j.foodchem.2017.06.150 | 1-8 |
| A | MITAR A y KARDUM J. Intensification of Mass Transfer in the Extraction Process with a Nanofluid Prepared in a Natural Deep Eutectic Solvent. Chemical Engineering And Technology, 2020, vol. 43 (11), pages 2286 - 2294, ISSN 0930-7516 (print) ISSN 1521-4125 (electronic), DOI:10.1002/ceat.202000097 | 1-8 |

☒ Further documents are listed in the continuation of Box C.      ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | "&" document member of the same patent family |

| Date of the actual completion of the international search | Date of mailing of the international search report |
|---|---|
| 18/11/2022 | **(23/11/2022)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | A. Polo Diez Telephone No. 91 3495524 |

Form PCT/ISA/210 (second sheet) (January 2015)

## INTERNATIONAL SEARCH REPORT

International application No.

PCT/ES2022/070561

C (continuation).                    DOCUMENTS CONSIDERED TO BE RELEVANT

| Category * | Citation of documents, with indication, where appropriate, of the relevant passages | Relevant to claim No. |
|---|---|---|
| A | CHANIOTI S. et al. Novel processes for the extraction of phenolic compounds from olive pomace and their protection by encapsulation. Molecules, 22/03/2021, vol. 26 (6), pages 1-18, ISSN 1420-3049 (Electronic, DOI: 10.3390/molecules26061781 pubmed:33810031 | 1-8 |
| A | FERNANDEZ-PRIOR M A et al. Deep eutectic solvents improve the biorefinery of alperujo by extraction of bioactive molecules in combination with industrial thermal treatments. Food And Bioproducts Processing, 2020, vol. 121, pages 131 - 142, ISSN 0960-3085 (print), DOI:10.1016/j.fbp.2020.02.001 | 1-8 |
| A | PANIC M. et al. Ready-to-use green polyphenolic extracts from food by-products. Food Chemistry, 2019, vol. 283, pages 628-636, ISSN 0308-8146(print) ISSN 1873-7072(electronic), DOI: 10.1016/j.foodchem.2019.01.061 | 1-8 |
| A | PLAZA A. et al. Obtaining Hydroxytyrosol from Olive Mill Waste Using Deep Eutectic Solvents and Then Supercritical $CO_2$. Waste And Biomass Valorization, 2019, vol. 11 (11), pagss 6273 -6284, ISSN 1877-2641 (print) ISSN 1877-265X (electronic), DOI: 10.1007/s12649-019-00836-1 | 1-8 |
| A | OBIED H K et al. Bioscreening of Australian olive mill waste extracts: Biophenol content, antioxidant, antimicrobial and molluscicidal activities. Food And Chemical Toxicology, 2007, vol. 45(7), pages 1238-1248, ISSN 0278-6915(print) ISSN 1873-6351(electronic), DOI: 10.1016/j.fct.2007.01.004 | 1-8 |
| A | EP 3468941 A1 (UNIV DO PORTO) 17/04/2019, claims. | 1-8 |
| A | MELGAR R et al. Bioconversion of wastes from olive oil industries by vermicomposting process using the epigeic earthworm *Eisenia andrei*. Journal Of Environmental Science And Health - Part B Pesticides, Food Contaminants, And Agricultural Wastes, 2009, vol. 44 (5), pages 488 - 495, ISSN 0360-1234 (print) ISSN 1532-4109 (electronic), DOI: 10.1080/03601230902935444 | 1-8 |
| A | TORTOSA G et al. The production of comercial organic amendments and fertilisers by composting of two-phase olive mill waste. Journal of Cleaner Production, 2011, vol. 26, pages 48 - 55, ISSN 0959-6526 (print), DOI: 10.1016/j.jclepro.2011.12.008 | 1-8 |

Form PCT/ISA/210 (continuation of second sheet) (January 2015)

## INTERNATIONAL SEARCH REPORT

Information on patent family members

International application No.

PCT/ES2022/070561

| Patent document cited in the search report | Publication date | Patent family member(s) | Publication date |
|---|---|---|---|
| WO2021180996 A1 | 16.09.2021 | ES2927301 A2 | 03.11.2022 |
| EP3468941 A1 | 17.04.2019 | MA45247 A | 17.04.2019 |
| | | US2019153352 A1 | 23.05.2019 |
| | | US10479958 B2 | 19.11.2019 |
| | | WO2017212450 A1 | 14.12.2017 |

Form PCT/ISA/210 (patent family annex) (January 2015)

## INTERNATIONAL SEARCH REPORT

International application No.

PCT/ES2022/070561

CLASSIFICATION OF SUBJECT MATTER

*B01D11/02* (2006.01)
*A23L33/105* (2016.01)
*A01N65/08* (2009.01)
*C05F5/00* (2006.01)
*A01K67/033* (2006.01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **E. BENITEZ, E ; SAINZ, H ; MELGAR, R ; NOGALES, R.** Vermicomposting of a lignocellulosic waste from olive oil industry: A pilot scale study. *Waste Manage Res.,* 2002, 134-142

- **VENIERI, D ; ROUVALIS, A. ; ILIOPOULOU-GEORGUDAKI, J.** Microbial and toxic evaluation of raw and treated olive mill wastewaters. *J Chem Tech & Biotech,* 2010, vol. 85, 1380-1388

- **S. DEMERCHE ; M. NADOUR ; C. LARROCHEB ; F. MOULTI-MATIA ; P. MICHAUDB.** Olive mill wastes: Biochemical characterizations and valorization Strategies. *Process Biochem,* 2013, vol. 48, 1532-1552

- **SILVA RFM ; POGAČNIK L.** Polyphenols from Food and Natural Products: Neuroprotection and Safety. *Antioxidants (Basel).,* 10 January 2020, vol. 9 (1

- **SHAHIDI, F. ; NACZK, M.** Phenolics in Food and Nutraceuticals. CRC Press, 2004

- **SAJID MAQSOOD ; SOOTTAWAT BENJAKUL ; FEREIDOON SHAHIDI.** Emerging Role of Phenolic Compounds as Natural Food Additives in Fish and Fish Products. *Critical Reviews in Food Science and Nutrition,* 2013, vol. 53 (2

- **BALASUBRAMANIAN, P. ; KARTHICKUMAR, P.** Biofertilizers and biopesticides: A holistic approach for sustainable agriculture. *Sustainable utilization of natural resources,* 2017, 256-284

- **KULKARNI, S.** Sustainable agrochemicals for conservation of agriculture and climate change. *Conservation agriculture: An approach to combat climate change in indian himalaya,* 2016, 135-157

- **BRENES, M ; MEDINA, E. ; GARCIA, A. ; ROMERO, C. ; CASTRO, A.** Olives and olive oil compounds active against pathogenic microrganisms. *Health and disease prevention,* 2010, vol. 109, 1013-1019

- **SÁNCHEZ, P ; RUIZ, M.V.** Production of pomace olive oil. *Grasas y aceites,* 2006, vol. 57, 47-55

- **MEDINA, E ; ROMERO, C ; SANTOS, B. ; CASTRO, A. ; GARCIA, A. ; ROMERO, F ; ROMERO, F. ; BRENES, M.** Antimicrobial activity of olive solutions from stored alpeorujo against plant pathogenic microorganisms. *J. Agric. Food Chem.,* 2011, vol. 59, 6927-6932

- **DAI, Y. ; WITKAMP, G. J ; VERPOORTE, R. ; CHOI, Y. H.** Natural Deep Eutectic Solvents as a New Extraction Media for Phenolic Metabolites in Carthamus tinctorius. L. *Anal. Chem.,* 2013, vol. 85, 6272-6278

- **RADOŠEVIĆ, K ; BUBALO, M. C ; SRČEK, V. G ; GRGAS, D ; DRAGIČEVIC, T. L. ; REDOVNIKOVIC, I. R.** Evaluation of toxicity and biodegradability of choline chloride based deep eutectic solvents. *Ecotoxicol Environ Saf.,* 2015, vol. 112, 46-53

- **GARCIA, A. ; RODRIGUEZ-JUAN, E. ; RODRÍGUEZ-GUTIÉRREZ, G. ; RIOS, J. J. ; FERNÁNDEZ-BOLAÑOS, J.** Extraction of phenolic compounds from virgin olive oil by deep eutectic solvents (DESs). *Food Chem.,* 2016, vol. 197, 554-561

- **GARCIA BORREGO, A ; RODRIGUEZ-JUAN, E ; FERNANDEZ-BOLAÑOS, J.** Extraction of phenolic compounds from olive pomace by deep eutectic solvents (DESs). *Trends in Green chem.,* 2017, vol. 3, 3

- **SÁNCHEZ, P ; RUIZ, M.V.** Production of pomace olive oil. *Grasas y aceites,* 2006, vol. 57, 47-55

- **TORTOSA, G. ; ALBUQUERQUE, J.A. ; AIT-BADDI, G ; CEGARRA, J.** The production of commercial organic amendments and fertilisers by composting of two-phase olive mill waste ("alpeorujo). *Journal of Cleaner Production,* 2012, vol. 26, 48-55

- **MELGAR, R ; BENITEZ, E ; NOGALES, R.** Bioconversion of wastes from olive oil industries by vermicomposting process using the epigeic earthworm Eisenia andrei. *Journal of Environmental Science and Health, Part B,* 2009, vol. 44, 488-495

- **NOGALES, R. ; ROMERO, E. ; FERNANDEZ-GÓMEZ, M. J.** Vermicompost: Processes, products and applications. Mundi-Prensa, 2014

- **BRENES, M ; GARCIA, A ; DE LOS SANTOS, B ; MEDINA, E ; ROMERO, C. ; CASTRO, A. ; ROMERO, F.** Olive glutaraldehyde-like compounds against plant pathogenic bacteria and fungi. *Food Chemistry,* 2010, vol. 125, 1262-1266

- **JARAMILLO, S. ; MURIANA F.J.G. MURIANA ; GUILLEN, R ; JIMENEZ-ARAUJO, A ; RODRIGUEZ-ARCOS, R. ; LOPEZ, S.** Saponins from edible spears of wild asparagus inhibit AKT, 2 p70S6K, and ERK signalling, and induce apoptosis through 3 G0/G1 cell cycle arrest in human colon cancer HCT-116 cells. *Journal of Functional Foods,* 2016, vol. 26, 1-10